# EUROPÄISCHE PATENTANMELDUNG

(11) **EP 0 818 367 A2**
(43) Veröffentlichungstag der Anmeldung: **14.01.1998**
(21) Anmeldenummer: 97109074.1
(22) Anmeldetag: 05.06.1997
(51) Int. Cl.: B60R 21/26

(54) **Steuerverfahren für eine Sicherheitseinrichtung eines Fahrzeugs**

(30) Priorität: 27.06.1996 DE 19625890
(71) Anmelder: Bayerische Motoren Werke Aktiengesellschaft, 80788 München (DE)
(72) Erfinder: Griessbach, Robert, 85635 Höhenkirchen-Siegertsbrunn (DE)

(57) **Zusammenfassung**

Bei einem Steuerverfahren für eine Sicherheitseinrichtung eines Fahrzeugs, bei dem die Wirkungsweise der Sicherheitseinrichtung an das Gewicht des Fahrzeugbenutzers angepaßt und bei der ein berührungslos arbeitender Sensor auf den Fahrzeugbenutzer ausgerichtet ist, werden vor dem Auslösen der Einrichtung Körpermaße des Fahrzeugsbenutzers mittels des Sensors aufgenommen und aus den Körpermaßen mittels gespeicherter Daten wird das Gewicht des Fahrzeugbenutzers angenähert bestimmt.

## Beschreibung

Die Erfindung bezieht sich auf ein Steuerverfahren mit den Merkmalen des Oberbegriffs von Patentanspruch 1.

Bei den Sicherheitseinrichtungen kann es sich beispielsweise um einen Frontal- bzw. Seiten-Airbag, einen Gurtstrammer oder aber auch um Aufschlagselemente handeln, die im Bewegungsbereich des Fahrzeugbenutzers angeordnet sind. Durch die Anpassung der Wirkungsweise an das Gewicht des Fahrzeugbenutzers soll die Verletzungsgefahr inbesondere bei einer extremen Belastung des Fahrzeugbenutzers durch die Sicherheitseinrichtung selbst minimiert werden.

Aus der DE 41 12 579 A1 ist ein Steuerverfahren der eingangs genannten Art bekannt, bei dem mit Hilfe eines berührungslos arbeitenden Sensors, vorzugsweise einer optischen Kamera, vor dem Auslösen der Sicherheitseinrichtung die Sitzhaltung und im Crashfall die Bewegungsbahn des Fahrzeugbenutzers bestimmt und die Wirkungsweise der Sicherheitseinrichtungen entsprechend gesteuert wird. Zusätzlich ist dabei die Möglichkeit vorgesehen, das Gewicht des Fahrzeugbenutzers zu berücksichtigen. Hierzu wird das Gewicht mit Hilfe einer Handeingabeeinrichtung vor Antritt der Fahrt eingegeben. Dabei kann es zu Fehleingaben kommen. Ebenso kann die Eingabe des Gewichts unterlassen werden. Dadurch ist die Wirkungsweise des bekannten Steuerverfahrens nicht zuverlässig, da sie auf fehlenden bzw. unrichtigen Daten über das Gewicht des Fahrzeugbenutzers beruhen kann und möglicherweise zu einer Wirkungsweise der Sicherheitseinrichtung führt, die dem Gewicht des Fahrzeugbenutzers gerade nicht angepaßt ist. Damit kann die vermeintliche Wirkungsverbesserung der Sicherheitseinrichtung ins Gegenteil verkehrt werden.

Der Erfindung liegt die Aufgabe zugrunde, ein Steuerverfahren der eingangs genannten Art zu schaffen, bei dem die Zuverlässigkeit gerade hinsichtlich der Gewichtsbestimmung des Fahrzeugbenutzers wesentlich verbessert ist.

Die Erfindung löst diese Aufgabe durch die kennzeichnenden Merkmale des Patentanspruchs 1.

Im Gegensatz zu den bekannten Verfahren wird nunmehr das Gewicht zwar nicht mehr exakt berücksichtigt, sondern nur näherungsweise abgeschätzt. Andererseits erfolgt diese Abschätzung bei jeder Benutzung des Fahrzeugs und selbsttätig ohne das Zutun des Fahrzeugbenutzers. Der berührungslose Sensor, bei dem es sich wie an sich bekannt um eine optische Kamera oder aber auch um eine Infrarot-Kamera handeln kann, dient nicht oder nicht nur der Erkennung der Sitzhaltung des Fahrzeugbenutzers, sondern dient als Hilfsmittel zur Bestimmung des Gewichts. Die hierfür erforderlichen Daten liegen gespeichert als Tabellenwerte beispielsweise in einem Steuergerät für die Sicherheitseinrichtung(en) vor und können ohne weiteres für die Gewichtsbestimmung herangezogen werden.

Ergänzend kann wie an sich ebenfalls aus der DE 4112579 A1 die Neigung der Sitzlehne berücksichtigt werden, um die Gewichtschätzung zu präzisieren. Anhand der Lehnenneigung und der Abstandsdaten von Punkten, die bei einem Sagittalschnitt in der Körperoberfläche liegen, läßt sich das Gewicht des Fahrzeugbenutzers mit hinreichender Genauigkeit bestimmen. Dadurch kann ein separater Geber für das Auflagegewicht des Fahrzeugbenutzers entfallen. Probleme, die sich aus unterschiedlichen Gewichtswerten bei unterschiedlicher Sitz- Lehneneigung ergeben, lassen sich dadurch ebenfalls eliminieren. Auch die Zuverlässigkeit des Gesamtsystems ist, bedingt durch den Wegfall einer separaten Gewichtssensorik, deutlich erhöht.

## Patentansprüche

1. Steuerverfahren für eine Sicherheitseinrichtung eines Fahrzeugs, bei dem die Wirkungsweise der Sicherheitseinrichtung an das Gewicht des Fahrzeugbenutzers angepaßt und bei der ein berührungslos arbeitender Sensor auf den Fahrzeugbenutzer ausgerichtet ist,
dadurch gekennzeichnet,
daß vor dem Auslösen der Einrichtung Körpermaße des Fahrzeugsbenutzers mittels des Sensors aufgenommen werden und daß aus den Körpermaßen mittels gespeicherter Daten das Gewicht des Fahrzeugbenutzers angenähert bestimmt wird.

2. Steuerverfahren nach Anspruch 1,
dadurch gekennzeichnet,
daß als Sensor eine optische Kamera verwendet wird.

3. Steuerverfahren nach Anspruch 1,
dadurch gekennzeichnet,
daß als Sensor eine Infrarot-Kamera verwendet wird.
